# EUROPEAN PATENT APPLICATION

(11) **EP 2 128 171 A1**
(43) Date of publication of application: **02.12.2009**
(21) Application number: 08738668.6
(22) Date of filing: 19.03.2008
(51) Int. Cl.: C07J 53/00, A61K 31/56, A61P 3/00, A61P 7/00, A61P 39/02, A61P 43/00

(54) **AGENT FOR PREVENTION OR TREATMENT OF IRON OVERLOAD**

(30) Priority: 20.03.2007 JP 2007071877
(71) Applicant: Meiji Seika Kaisha Ltd., Tokyo 104-8002 (JP)
(72) Inventor: KAGEHARA, Hideaki, Tokyo 104-8002 (JP); NISHIYAMA, Shoji, Tokyo 104-8002 (JP)
(74) Representative: Cohausz & Florack
(86) International application number: PCT/JP2008/055060
(87) International publication number: WO 2008/123093

(57) **Abstract**

Disclosed is an agent for prevention or treatment of iron overload disorders, comprising 22β-methoxyolean-12-ene-3β,24(4β)-diol or a pharmacologically acceptable salt thereof.

## Description

### TECHNICAL FIELD

The present invention relates to an agent for prevention or treatment of iron overload disorders, comprising 22β-methoxyolean-12-ene-3β,24(4β)-diol or a pharmacologically acceptable salt thereof.

### BACKGROUND ART

Chronic iron overload is characterized by elevated focal or generalized iron deposition in tissues. It is generally termed hemosiderosis in histological examination, but excess iron deposition accompanied by tissue damage (or a total body iron content of at least 5 g) is termed hemochromatosis (see non-patent literature 1).

Iron overload disorder is classified by cause. More particularly, iron overload is mainly classified into elevated iron uptake from diet (hereditary hemochromatosis, chronic liver diseases, porphyria cutanea tarda, atransferrinemia, oral administration of excess iron, or the like), excess iron load by parenteral administration (transfusional iron overload, intravenous injection of excess iron, or the like), and diseases caused by both thereof (hereditary tyrosinemia, cerebrohepatorenal syndrome, neonatal hemochromatosis, idiopathic pulmonary hemosiderosis, renal hemosiderosis, or the like). Idiopathic hemochromatosis and thalassemia are well-known as a congenital disease. Acquired iron overload includes intravenous injection of excess iron, and transfusional iron overload. See non-patent literature 2.

Iron overload disorders result in organ damage, in particular the liver, heart, and/or pancreas, and progresses to a fatal condition. Elevated iron storage in the whole body, excess iron deposition in parenchymal cells of the heart, pancreas, liver, and other organs in the form of ferritin and hemosiderin, and morphologic and functional disorder in organs and regions where excess iron is deposited, are observed as general symptoms. An injection of an iron chelator is an example of a commonly used therapeutic agent for transfusional iron overload disorders. However, this injection is not suitable for outpatients, because it must be administered on consecutive days for a long time to obtain sufficient effects, and thus, an iron chelator which can be orally administered has been recently developed. Under these circumstances, development of medicaments effective in treating iron overload disorders without adverse effects is desired.

22β-Methoxyolean-12-ene-3β,24(4β)-diol is a compound of the following formula, and is known as a compound having an inhibitory effect of hepatocyte dysfunction with a high level of safety (see patent literatures 1 and 2).

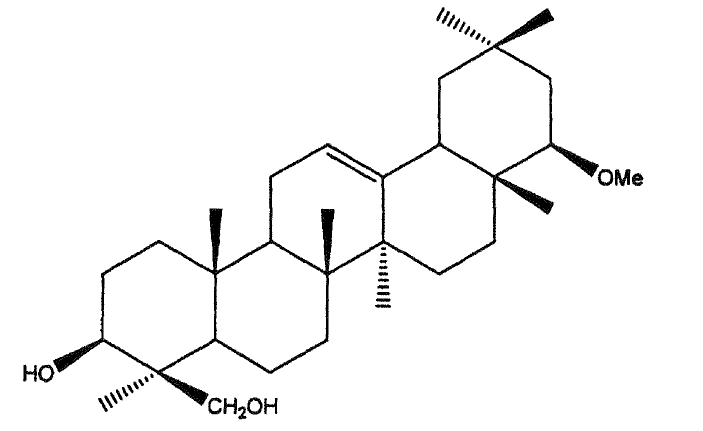

[patent literature 1] WO 97/03088
[patent literature 2] Japanese Patent No. 3279574
[non-patent literature 1] Mark H. Beers and one other, Masanori FUKUSHIMA, "The Merck Manual, 17th ed. Japanese ed.", Nikkei Business Publications, Inc., December 10, 1999, p.883-885
[non-patent literature 2] Masaaki TAKAHASHI, "Transfusional Iron Overload disorder and Iron Chelation Therapy", Saishin Igaku, March 2006, vol.61, no.3, p.453-457

### DISCLOSURE OF THE INVENTION

### PROBLEMS TO BE SOLVED BY THE INVENTION

To solve the above problems, the present inventors conducted intensive studies on medicaments effective in the treatment or prevention of iron overload disorders, and found that a serum ferritin level could be reduced by administering 22β-methoxyolean-12-ene-3β,24(4β)-diol, and that this compound could be an excellent pharmaceutical composition for the prevention or treatment of iron overload disorders, and completed the present invention.

### MEANS FOR SOLVING THE PROBLEMS

The present invention relates to
[1] an agent for prevention or treatment of iron overload disorders, comprising 22β-methoxyolean-12-ene-3β,24(4β)-diol or a pharmacologically acceptable salt thereof,
[2] the agent of [1], wherein iron overload disorders are hemochromatosis,
[3] use of 22β-methoxyolean-12-ene-3β,24(4β)-diol or a pharmacologically acceptable salt thereof in the manufacture of an agent for prevention or treatment of iron overload disorders,
[4] the use of [3], wherein iron overload disorders are hemochromatosis,
[5] 22β-methoxyolean-12-ene-3β,24(4β)-diol or a pharmacologically acceptable salt thereof for prevention or treatment of iron overload disorders,
[6] the compound of [5], wherein iron overload disorders are hemochromatosis,
[7] a method for prevention or treatment of iron overload disorders, comprising administering to a subject in need thereof 22β-methoxyolean-12-ene-3β,24(4β)-diol or a pharmacologically acceptable salt thereof in an amount effective therefor,
[8] the method of [7], wherein iron overload disorders are hemochromatosis.

### EFFECTS OF THE INVENTION

The prophylactic or therapeutic agent of the present invention exhibits high prophylactic or therapeutic effects on iron overload disorders by administering 22β-methoxyolean-12-ene-3β,24(4β)-diol or a pharmacologically acceptable salt thereof.

### BRIEF DESCRIPTION OF THE DRAWINGS

[Figure 1] Figure 1 is a graph showing the time course of serum ferritin levels (average) after the oral administration of 22β-methoxyolean-12-ene-3β,24(4β)-diol (50 mg/day or 200 mg/day).
[Figure 2] Figure 2 is a graph showing the time course of serum ferritin levels (median) after the oral administration of 22β-methoxyolean-12-ene-3β,24(4β)-diol (50 mg/day or 200 mg/day).

### BEST MODE FOR CARRYING OUT THE INVENTION

The term "iron overload disorders" as used herein means a condition in which a storage iron level and a serum iron level are higher than normal levels. Examples of iron overload disorders in the present invention include focal hemosiderosis, hereditary hemochromatosis, secondary hemosiderosis, secondary hemochromatosis, and cryptogenic iron overload (an increase in iron storage caused by hepatic parenchymal disease, nonalcoholic fatty liver, or chronic hepatitis C), preferably hemochromatosis.

The term "hemochromatosis" as used herein refers to a disease accompanied by tissue damage caused by excess iron deposition, or having a total body iron content of 5 g or more. Hemochromatosis includes hereditary iron overload disorders and nonhereditary iron overload disorders. Nonhereditary iron overload disorders are caused by transfusional iron overload, or decreased iron availability due to a disorder of erythrocyte production, and is also called secondary hemochromatosis. Hemochromatosis is a disorder of iron metabolism characterized by excess absorption of ingested iron, saturation of iron-binding protein, and deposition of hemosiderin in tissue, particularly in the liver, pancreas, and skin. Cirrhosis of the liver, diabetes (bronze diabetes), bronze pigmentation of the skin, and eventually heart failure may occur. Hemochromatosis also can result from administration of large amounts of iron orally, parenterally, or in forms of blood transfusion therapy (STEDMAN'S Medical Dictionary, 4th Edition, 1998, Medical View Co., Ltd.).

22β-Methoxyolean-12-ene-3β,24(4β)-diol, which may be used as the active ingredient for the prophylactic or therapeutic agent of the present invention, is a known compound, and can be obtained by, for example, the method described in Example 22 (Compound 27) of WO97/03088. 22β-Methoxyolean-12-ene-3β,24(4β)-diol can be easily converted into a salt thereof by reacting the compound with a pharmaceutically acceptable base. Preferable bases include inorganic bases such as sodium hydroxide, potassium hydroxide, aluminum hydroxide, sodium carbonate, potassium carbonate, and sodium hydrogen carbonate, and organic bases such as piperazine, morpholine, piperidine, ethylamine, and trimethylamine.

In general, 22β-methoxyolean-12-ene-3β,24(4β)-diol or a pharmacologically acceptable salt thereof may be orally administered as a conventional pharmaceutical formulation, such as capsules, microcapsules, tablets, granules, fine granules, powders, or the like. Further, it may be parenterally administered (for example, by intravenous injection, intramuscular injection, subcutaneous administration, intraperitoneal administration, rectal administration, percutaneous administration) as a conventional pharmaceutical formulation, such as injections (intravenous, intramuscular, or the like) or the like. These formulations can be prepared by a conventional method using an excipient, a filler, a binder, a wetting agent, a disintegrating agent, a surfactant, a lubricant, a dispersing agent, a buffer, a preservative, a solubilizer, an antiseptic, a flavor, a soothing agent, a stabilizer, and the like. Examples of the above additives which are nontoxic and suitable for the preparations include lactose, fructose, glucose, starch, gelatin, magnesium carbonate, synthetic magnesium silicate, talc, magnesium stearate, methylcellulose, carboxymethylcellulose or a salt thereof, gum arabic, polyethylene glycol, syrup, vaseline, glycerin, ethanol, propylene glycol, citric acid, sodium chloride, sodium sulfite, sodium phosphate, and the like. As described above, 22β-methoxyolean-12-ene-3β,24(4β)-diol or a pharmacologically acceptable salt thereof may be administered in the form of a pharmaceutical composition containing a pharmaceutically acceptable carrier or diluent.

In the prophylactic or therapeutic agent of the present invention, the form, the route of administration, the dose, the period of administration, and the like of 22β-methoxyolean-12-ene-3β,24(4β)-diol may be appropriately selected in accordance with, for example, the weight, the age, the symptoms, and the like of a patient. For example, a daily dose of 1 to 1000 mg is orally or parenterally administered as a single dose or divided into multiple doses. Preferably, a daily dose of 25 to 800 mg is divided into two doses, which are orally or parenterally administered.

Serum ferritin, serum iron, total iron binding capacity (TIBC), and the like are known as laboratory findings useful for diagnosis of iron overload disorders. Increased iron load results in proportionally increased serum ferritin. Serum ferritin and liver MRI are recommended for monitoring of iron overload disorders because of ease and imposing a light load on the patient (Saishin Igaku, vol.61, no.3, p.453-457, 2006).

### EXAMPLES

The present invention now will be further illustrated by, but is by no means limited to, the following Example.

### Example 1

To patients suffering from chronic hepatitis C, 22β-methoxyolean-12-ene-3β,24(4β)-diol was orally administered for 24 weeks, at a daily dose of 50 mg/day or 200 mg/day. The administration of 22β-methoxyolean-12-ene-3β,24(4β)-diol in each administration group was carried out by orally administering half of each daily dose twice a day after eating breakfast and dinner. For example, in the 50 mg/day administration group, each day 25 mg of the drug was orally administered after breakfast and 25 mg of the drug was orally administered after dinner. Immediately after the beginning of the administration, and after 2, 4, 8, 12, 16, 20, and 24 weeks from the beginning of the administration, serum samples were collected to determine serum ferritin contained in the samples. The numbers of patients for collecting serum samples are 49, 48, 47, 44, 45, 44, and 40 in the 50 mg/day administration group, and 45, 44, 43, 43, 43, 41, and 39 in the 200 mg/day administration group, after 2, 4, 8, 12, 16, 20, and 24 weeks, respectively. Serum ferritin was determined by a chemiluminescence immunoassay (CLIA) with reference to a method described in a protocol attached to Chemilumi ACS-Ferritin II (Bayer Medical).

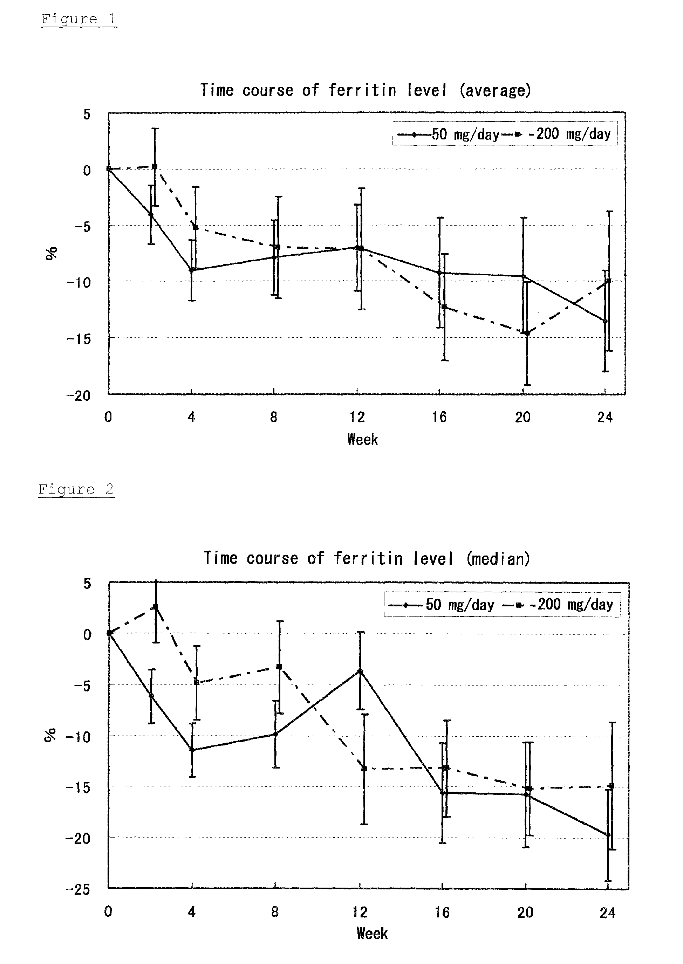

Each serum ferritin level contained in each serum sample collected was indicated as percentage, with the serum ferritin level before the administration of 22β-methoxyolean-12-ene-3β,24(4β)-diol regarded as 0, and an average and a median of all patients were calculated. The results are shown in Figures 1 and 2.

As a result, it was confirmed that serum ferritin was lowered by the administration of 22β-methoxyolean-12-ene-3β,24(4β)-diol and this compound could be used as a potent active ingredient of a pharmaceutical composition for prevention or treatment of iron overload disorders.

### INDUSTRIAL APPLICABILITY

According to the present invention, an agent for prevention or treatment of iron overload disorders, comprising 22β-methoxyolean-12-ene-3β,24(4β)-diol or a pharmacologically acceptable salt thereof can be provided.
Although the present invention has been described with reference to specific embodiments, various changes and modifications obvious to those skilled in the art are possible without departing from the scope of the appended claims.

## Claims

1. An agent for prevention or treatment of iron overload disorders, comprising 22β-methoxyolean-12-ene-3β,24(4β)-diol or a pharmacologically acceptable salt thereof.

2. The agent according to claim 1, wherein iron overload disorders are hemochromatosis.

3. Use of 22β-methoxyolean-12-ene-3β,24(4β)-diol or a pharmacologically acceptable salt thereof in the manufacture of an agent for prevention or treatment of iron overload disorders.

4. The use according to claim 3, wherein iron overload disorders are hemochromatosis.

5. 22β-Methoxyolean-12-ene-3β,24(4β)-diol or a pharmacologically acceptable salt thereof for prevention or treatment of iron overload disorder.

6. The compound according to claim 5, wherein iron overload disorders are hemochromatosis.

7. A method for prevention or treatment of iron overload disorders, comprising administering to a subject in need thereof 22β-methoxyolean-12-ene-3β,24(4β)-diol or a pharmacologically acceptable salt thereof in an amount effective therefor.

8. The method according to claim 7, wherein iron overload disorders are hemochromatosis.
